# FASCICULE DE BREVET EUROPEEN

(11) **EP 2 890 401 B1**
(45) Date de publication et mention de la délivrance du brevet: **28.10.2020**
(21) Numéro de dépôt: 13803222.2
(22) Date de dépôt: 30.08.2013
(51) Int. Cl.: A61K 47/34, A61K 31/135

(54) **FORMULATION GELIFIANTE A BASE DE KETAMINE**
GELIERUNGSFORMULIERUNG MIT KETAMIN
GELLING FORMULATION CONTAINING KETAMINE

(30) Priorité: 31.08.2012 FR 1258146
(43) Date de publication de la demande: 08.07.2015
(73) Titulaire: Assistance Publique Hôpitaux De Paris, 75004 Paris 4 (FR)
(72) Inventeur: BOUDY, Vincent, 75013 Paris (FR); TIBI, Annick, 94220 Charenton (FR); D'HAYER, Benoît, 75019 Paris (FR); HUSSON, Marie-Caroline, 75005 Paris (FR); GRAFF DE FAGET, Sandrine, 78170 La Celle Saint Cloud (FR)
(74) Mandataire: Gevers & Orès
(86) Numéro de dépôt international: PCT/IB2013/058165
(87) Numéro de publication internationale: WO 2014/033680

(56) Documents cités:
- US-A1- 2003 185 761
- US-A1- 2010 015 263

## Description

L'invention concerne une formulation gélifiante à base de kétamine, son utilisation dans le traitement de la douleur, et son procédé de préparation et d'administration.

Le chlorhydrate de kétamine ou (RS)-2-(2-chlorophényl)-2-méthylamino-cyclohexan-1-one, un antagoniste non compétitif des récepteurs médullaires NMDA (N-méthyl-D-aspartate) post-synaptique au glutamate, est un produit utilisé en France en anesthésie humaine et vétérinaire. Il se caractérise par un effet anesthésique général et une action rapide. Il peut être utilisé comme agent anesthésique unique : particulièrement adapté aux interventions de courte durée, il permet également, grâce à des injections répétées ou à son utilisation en perfusion intraveineuse, d'obtenir une anesthésie prolongée durant plusieurs heures. Il peut être également utilisé soit comme inducteur d'anesthésie avant l'administration d'autres agents anesthésiques, soit comme potentialisateur d'agents anesthésiques de faible puissance, tel que le protoxyde d'azote.

Il est par ailleurs utilisé hors cadre de l'autorisation de mise sur le marché (AMM), en tant qu'agent analgésique, en particulier pour le traitement des douleurs aiguës, notamment en pédiatrie.

Les spécialités pharmaceutiques à base de kétamine actuellement sur le marché en France sont des solutions injectables destinées à une administration par voie intraveineuse ou intramusculaire, lesquelles sont difficiles d'accès chez l'enfant.

US2003/185761 A1 divulgue des sprays aérosols destinés à une application buccale comprenant un agent actif tel que la kétamine et un solvant polaire permettant une absorption rapide à travers les muqueuses de la cavité buccale

Il a maintenant été mis au point une nouvelle formulation permettant de pallier aux inconvénients des spécialités pharmaceutiques actuelles.

Plus particulièrement, l'invention concerne une formulation à base de kétamine, capable de façon avantageuse, d'être pulvérisée sous la forme d'un spray liquide dans la cavité buccale et de se gélifier instantanément au contact des muqueuses de la bouche, et de permettre une libération rapide et prolongée de la kétamine. Comparée à une solution, la présence d'une texture gel permet un contact prolongé de la kétamine avec la surface des muqueuses. Cette formulation offre plusieurs avantages : elle est tout d'abord facile à appliquer et permet d'augmenter d'une part, la surface d'échange dans la cavité buccale, lors de sa pulvérisation sous forme liquide, et d'autre part d'augmenter le temps de présence du principe actif dans la cavité buccale de par sa forme gel ; elle permet d'accéder à une meilleure biodisponibilité de la kétamine ; elle peut être par ailleurs aisément préparée et conditionnée pour être conservée dans des conditions stériles, et peut ne pas contenir d'agents de conservation. Enfin, cette formulation permet de prodiguer des premiers soins de façon rapide, sûre et économique, en particulier aux urgences chez l'enfant.

L'objet de la présente invention est défini par les revendications 1 à 9.

Ainsi, selon un premier aspect, la description concerne une formulation gélifiante comprenant de la kétamine ou un sel pharmaceutiquement acceptable de celle-ci, un poloxamer, et de l'eau.

Par « formulation gélifiante », on entend, au sens de la présente description, une formulation capable de se gélifier, en particulier dans laquelle la concentration du ou des poloxamers est suffisante pour permettre à la solution aqueuse de se gélifier à une température donnée, notamment au contact de la peau, notamment des muqueuses de la cavité buccale, en particulier les muqueuses de la langue et des joues.

La kétamine est le composé de structure suivante :

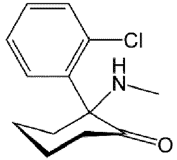

Selon un mode de réalisation préféré, les formulations gélifiantes selon la présente demande comprennent un sel pharmaceutiquement acceptable de la kétamine, en particulier le chlorhydrate de kétamine.

La kétamine peut être utilisée sous sa forme racémique, ou sous une forme énantiomériquement enrichie. De préférence, l'énantiomère S-(+) de la kétamine, également dénommé eskétamine, ou un sel pharmaceutiquement acceptable de celui-ci est utilisé.

Les formulations selon la présente demande comprennent de préférence de 2 à 20 g/100 mL de kétamine ou d'un sel pharmaceutiquement acceptable de celle-ci, plus préférentiellement environ de 2 à 10 g/100 mL de formulation, et notamment environ 8 g/100 mL de kétamine

Tel qu'on l'emploie ici, le terme « poloxamer » désigne un copolymère organisé en tri-blocs comprenant ou consistant en une chaîne centrale de polyoxypropylène (encore appelée polypropylène glycol, POP) greffée de part et d'autre par une chaîne de polyoxyéthylène (encore appelé polyéthylène glycol, POE). Les poloxamers sont généralement désignés par la lettre « P » (pour poloxamer) suivie par trois chiffres : les deux premiers chiffres multipliés par 100 donnent la masse moléculaire du cœur de polyoxypropylène, et le dernier chiffre multiplié par 10 donne le pourcentage de la teneur en polyoxéthylène. A titre d'exemple, P407 correspond à un poloxamer dont le cœur en polyoxypropylène a une masse moléculaire de 4000 g/mol et une teneur en polyoxyéthylène de 70%.

Les poloxamers utiles selon la description sont des poloxamers thermosensibles, qui en fonction de leur concentration en solution, sont à l'état liquide à température ambiante, et notamment entre +8°C et +33°C, et à l'état de gel à une température supérieure ou égale à la température de gélification (T*_{g}*), notamment dans des conditions physiologiques, et en particulier entre +30°C et +40°C.

Les températures de gélification des poloxamers (T*_{g}*) peuvent être déterminées selon des méthodes conventionnelles ou sont disponibles dans des ouvrages de référence tels que le *Handbook of Pharmaceutical Excipients.*

Plus particulièrement, ces poloxamers sont présents dans une concentration suffisante dans la solution aqueuse de kétamine ou de l'un de ses sels pharmaceutiquement acceptable pour permettre sa gélification lorsque la température est supérieure ou égale à leur température de gélification (T*_{g}*), notamment dans des conditions physiologiques.

De préférence, la formulation gélifie à une température comprise entre +30°C et +37°C, plus préférentiellement au contact de la peau ou des muqueuses, notamment à une température comprise entre +31°C et +34°C.

Les formulations selon la présente demande comprennent de 15 à 20 g/100 mL de poloxamer, de préférence de 16 à 19 g/100 mL. Comme exemple, on peut citer notamment le poloxamer 407, le poloxamer 188 ou un mélange de ceux-ci.

Les formulations selon la présente demande peuvent comprendre en outre un ou plusieurs excipients pharmaceutiquement acceptables choisis parmi les agents mucoadhésifs, les agents de masquage de goût, les agents acide, les agents basique, les agents de conservation et le chlorure de sodium ou le chlorure de potassium.

Comme exemples d'agent muco-adhésif, on peut citer notamment les alginates (par exemple Kelton®, Manugel LBA®, Satialgine®), les gommes gellane (par exemple Gelzan®), les gommes xanthane (par exemple Satiaxane®), la gomme adragante, la gomme Karaya, les carraghénanes (par exemple Satiagel®), les pectines, les chitosans (par exemple Chitoclear®), l'acide hyaluronique et ses sels, les polymères de l'acide acrylique (par exemple Carbopol®, Noveon®), les copolymères de méthylvinyléther et de l'acide maléique (par exemple Gantrez®), la poly(N-vinylpyrrolidone) et ses dérivés (par exemple Kollidon 90F®), ainsi que les dérivés cellulosiques (HPMC, HPC, HEC, CMCNa).

Comme exemples d'agent de masquage de goût, on peut citer notamment les édulcorants pauvres en calorie tels que le sucralose, et la stévia et plus particulièrement de son extrait, le rébaudioside A à 97% (par exemple Rebaten 97®). Des arômes tels que tutti frutti, fraise, framboise, caramel, cola, agrume (orange, citron) peuvent également être utilisés pour masquer le goût de la formulation.

Comme exemples d'agent acide, utiles pour ajuster le pH de la solution, on peut citer notamment l'acide chlorhydrique, l'acide acétique, l'acide borique.

Comme exemples d'agent basique, également utiles pour ajuster le pH de la solution, on peut citer des solutions de soude et de potasse.

Comme exemples d'agent de conservation, on peut citer notamment l'acide sorbique, l'acide borique.

Selon un mode de réalisation, les formulations de la description comprennent environ 17 g/100 mL de poloxamer 407, et entre 0,04 et 2 g/100 mL d'agent mucoadhésif comme à titre d'exemple les alginates de sodium, les gommes xanthane, les carraghénanes.

De manière avantageuse, les formulations selon la description sont stériles et peuvent être préparées par filtration stérilisante, par radiostérilisation, ou bien dans des conditions aseptiques. Plus particulièrement, cette solution peut être préparée et/ou conservée dans des conditions stériles dans un flacon muni d'un pulvérisateur adapté.

Selon un autre aspect, la description concerne une composition pharmaceutique comprenant ou consistant en une formulation gélifiante telle que définie ci-dessus.

Selon encore un autre aspect, la description concerne une formulation gélifiante telle que définie ci-dessus pour son utilisation dans le traitement des douleurs, destinée notamment à être administrée dans la cavité buccale, par voie sublinguale.

De façon préférée, la formulation selon la présente demande est pulvérisée dans la cavité buccale, et forme instantanément un gel sur la zone d'application, préférentiellement sublinguale.

Selon un autre aspect, la description concerne un procédé de préparation d'une formulation gélifiante telle que définie ci-dessus, ledit procédé comprenant les étapes de :
i) Dissolution à froid du poloxamer dans l'eau, notamment à une température comprise entre +8°C et +16°C ;
ii) Ajout et dissolution d'un ou de plusieurs agents mucoadhésifs, de préférence à une température comprise entre +40°C et +70°C;
iii) Ajout et dissolution de la kétamine ou d'un sel de celle-ci dans la solution obtenue à l'étape i), de préférence à température ambiante, notamment à une température comprise entre +15°C et +25°C ; et
iv) Récupération de la formulation obtenue.

Selon une alternative, le procédé comprend les étapes de :
i) Dissolution de la kétamine ou d'un sel de celle-ci dans l'eau, de préférence à température ambiante, notamment à une température comprise entre +15°C et +25°C ;
ii) Ajout et dissolution d'un ou de plusieurs agents mucoadhésifs, de préférence à une température comprise entre +40°C et +70°C ;
iii) Ajout et dissolution du poloxamer dans la solution obtenue à l'étape i) préalablement refroidie, notamment à une température comprise entre +8 et +16°C ;et
iv) Récupération de la formulation obtenue.

### Définitions

Tel qu'on l'utilise dans la présente description, le terme « environ » se réfère à un intervalle de valeurs de ± 10 % d'une valeur spécifique. A titre d'exemple, l'expression « 17 g/100 mL environ » comprend les valeurs de 17 g/100 mL ± 10 %, soit les valeurs de 15,3 g/100 mL à 18,7 g/100 mL.

Au sens de la présente description, les ratios x g/100 mL se réfèrent à des ratios en poids par rapport à 100 mL de formulation finale à l'état liquide, sauf indication contraire.

Tel qu'on l'entend ici, les plages de valeur sous forme de « x-y » ou « de x à y » ou « entre x et y » incluent les bornes x et y ainsi que les entiers compris entre ces bornes. A titre d'exemple, « 1-6 », ou « de 1 à 6 » ou « entre 1 et 6 » désignent les entiers 1, 2, 3, 4, 5 et 6. Les modes de réalisations préférés incluent chaque entier pris individuellement dans la plage de valeur, ainsi que toute sous-combinaison de ces entiers. A titre d'exemple, les valeurs préférées pour « 1-6 » peuvent comprendre les entiers 1, 2, 3, 4, 5, 6, 1-2, 1-3, 1-4, 1-5, 2-3, 2-4, 2-5, 2-6, etc.

### EXEMPLES

### Exemple 1 : Préparation de formulations gélifiantes à base de chlorhydrate de kétamine (méthode A)

18 g de poloxamer 407 (Kolliphor P407®, BASF) ont été dissous à froid, dans 100 mL d'eau, puis 9,23g g de chlorhydrate de kétamine (soit 8 g de kétamine) ont été ajoutés et dissous en agitant pendant une heure environ.

La solution (**F1**) obtenue se gélifie au contact des muqueuses de la cavité buccale.

Les formulations **F2** à **F7** rapportées dans le tableau I ci-dessous ont été préparées selon le même mode opératoire. S'agissant des formulations **F3** et **F4**, l'agent mucoadhésif (alginate) a été préalablement ajouté dans la solution de poloxamer avant ajout de la kétamine à une température comprise entre +40°C et +70°C.

**Tableau I**

| **Formule No** | **Kétamine (g/100 mL)** | **Lutrol® F127 (Poloxamer 407) (g/100mL)** | **Manugel® LBA (Alginate) (g/100 mL)** |
|---|---|---|---|
| **F1** | 8 | 18 | - |
| **F2** | 8 | 19 | - |
| **F3** | 8 | 19 | 0,5 |
| **F4** | 8 | 19 | 1 |
| **F5** | 2 | 15 | |
| **F6** | 4 | 15 | |
| **F7** | 6 | 15 | |

### Exemple 2 : Préparation d'une formulation gélifiante à base de chlorhydrate de kétamine (méthode B)

9,23g g de chlorhydrate de kétamine (soit 8 g de kétamine) ont été dissous dans environ 100 ml d'eau en agitant.

Puis 18 g de poloxamer 407 (Kolliphor P407®, BASF) ont été dissous à froid.

La solution obtenue se gélifie au contact des muqueuses de la cavité buccale.

## Revendications

1. Formulation gélifiante pour son utilisation dans le traitement de la douleur, dans laquelle la formulation est mise en contact avec la surface des muqueuses de la bouche, ladite formulation comprenant de la kétamine ou un sel pharmaceutiquement acceptable de celle-ci, 15 à 20 g/100 mL d'un poloxamer, un ou plusieurs agents mucoadhésifs pharmaceutiquement acceptables et de l'eau.

2. Formulation gélifiante pour son utilisation selon la revendication 1, dans laquelle la kétamine est l'énantiomère S-(+) de la kétamine ou un sel pharmaceutiquement acceptable de celle-ci.

3. Formulation gélifiante pour son utilisation selon la revendication 1 ou 2, comprenant de 2 à 20 g/100 mL de kétamine ou d'un sel pharmaceutiquement acceptable de celle-ci, de préférence environ 8 g/100 mL de kétamine.

4. Formulation gélifiante pour son utilisation selon l'une quelconque des revendications précédentes, comprenant de 16 à 19 g/100 mL de poloxamer.

5. Formulation gélifiante pour son utilisation selon l'une quelconque des revendications précédentes, dans lequel le poloxamer est choisi parmi le poloxamer 407, le poloxamer 188 ou un mélange de ceux-ci.

6. Formulation gélifiante pour son utilisation selon l'une quelconque des revendications précédentes, comprenant en outre un ou plusieurs excipients pharmaceutiquement acceptables choisis parmi les agents de masquage de goût, les agents acide, les agents basique, les agents de conservation, le chlorure de sodium et le chlorure de potassium.

7. Formulation gélifiante pour son utilisation selon l'une quelconque des revendications précédentes, **caractérisée en ce qu'**elle est stérile.

8. Formulation gélifiante pour son utilisation selon l'une quelconque des revendications précédentes, **caractérisée en ce qu'**elle est destinée à une administration sublinguale.

9. Formulation gélifiante pour son utilisation selon l'une quelconque des revendications précédentes, **caractérisée en ce qu'**elle est destinée à être pulvérisée sous la forme d'un spray liquide dans la cavité buccale.

## Patentansprüche

1. Gelbildende Formulierung zur Verwendung bei der Behandlung von Schmerzen, wobei die Formulierung mit den Schleimhäuten des Munds in Kontakt gebracht wird, wobei die Formulierung Ketamin oder ein pharmazeutisch annehmbares Salz davon, 15 bis 20 g/100 ml ein Poloxamer, ein oder mehrere pharmazeutisch annehmbare mukoadhäsive Mittel und Wasser umfasst.

2. Gelbildende Formulierung zur Verwendung nach Anspruch 1, wobei das Ketamin das S-(+)-Enantiomer des Ketamins oder eines pharmazeutisch annehmbaren Salzes davon ist.

3. Gelbildende Formulierung zur Verwendung nach Anspruch 1 oder 2, umfassend von 2 bis 20 g/100 ml an Ketamin oder von einem pharmazeutisch annehmbaren Salz davon, vorzugsweise ungefähr 8 g/100 ml an Ketamin.

4. Gelbildende Formulierung zur Verwendung nach einem der vorstehenden Ansprüche, umfassend von 16 bis 19 g/100 ml an Poloxamer.

5. Gelbildende Formulierung zur Verwendung nach einem der vorstehenden Ansprüche, wobei das Poloxamer ausgewählt ist aus Poloxamer 407, Poloxamer 188 oder einem Gemisch von diesen.

6. Gelbildende Formulierung zur Verwendung nach einem der vorstehenden Ansprüche, weiter umfassend einen oder mehrere pharmazeutisch annehmbare Hilfsstoffe, ausgewählt aus Geschmacksmaskierungsmitteln, sauren Mitteln, basischen Mitteln, Konservierungsmitteln, Natriumchlorid und Kaliumchlorid.

7. Gelbildende Formulierung zur Verwendung nach einem der vorstehenden Ansprüche, **dadurch gekennzeichnet, dass** sie steril ist.

8. Gelbildende Formulierung zur Verwendung nach einem der vorstehenden Ansprüche, **dadurch gekennzeichnet, dass** sie für eine sublinguale Verabreichung bestimmt ist.

9. Gelbildende Formulierung zur Verwendung nach einem der vorstehenden Ansprüche, **dadurch gekennzeichnet, dass** sie dazu bestimmt ist, in der Form eines flüssigen Sprays in die Mundhöhle gesprüht zu werden.

## Claims

1. Gelling formulation for use thereof in pain treatment, wherein the formulation is put into contact with the surface of the mucous membranes of the mouth, said formulation comprising ketamine or a pharmaceutically acceptable salt thereof, 15 to 20g/100mL of a poloxamer, one or more pharmaceutically acceptable mucoadhesive agents and water.

2. Gelling formulation for use thereof according to claim 1, wherein ketamine is the enantiomer S-(+) of ketamine or a pharmaceutically acceptable salt thereof.

3. Gelling formulation for use thereof according to claim 1 or 2, comprising 2 to 20g/100mL of ketamine or a pharmaceutically acceptable salt thereof, preferably about 8g/100mL of ketamine.

4. Gelling formulation for use thereof according to any one of the preceding claims, comprising 16 to 19g/100mL of poloxamer.

5. Gelling formulation for use thereof according to any one of the preceding claims, wherein the poloxamer is selected from poloxamer 407, poloxamer 188 or a mixture thereof.

6. Gelling formulation for use thereof according to any one of the preceding claims, further comprising one or more pharmaceutically acceptable excipients selected from taste masking agents, acidic agents, basic agents, preservation agents, sodium chloride and potassium chloride.

7. Gelling formulation for use thereof according to any one of the preceding claims, **characterised in that** it is sterile.

8. Gelling formulation for use thereof according to any one of the preceding claims, **characterised in that** it is intended for sublingual administration.

9. Gelling formulation for use thereof according to any one of the preceding claims, **characterised in that** it is intended to be sprayed in the form of a liquid spray into the oral cavity.
